# EUROPEAN PATENT APPLICATION

(11) **EP 4 644 401 A1**
(43) Date of publication of application: **05.11.2025**
(21) Application number: 23912750.9
(22) Date of filing: 20.12.2023
(51) Int. Cl.: C07H 3/02, A23L 5/41, A23L 29/30

(54) **ALLULOSE SYRUP WITH IMPROVED PROPERTY**

(30) Priority: 29.12.2022 KR 20220189136
(71) Applicant: Samyang Corporation, Jongno-gu Seoul 03129 (KR)
(72) Inventor: PARK, Jiwon, Seongnam-si, Gyeonggi-do 13544 (KR); KIM, Goeun, Seongnam-si Gyeonggi-do 13434 (KR); KIM, Dohyun, Seongnam-si, Gyeonggi-do 13504 (KR); KIM, Minyoung, Suwon-si, Gyeonggi-do 16577 (KR); KIM, Minjeong, Yongin-si, Gyeonggi-do 16823 (KR); RYU, Kyunghun, Seongnam-si, Gyeonggi-do 13340 (KR); SA, Soonok, Yongin-si, Gyeonggi-do 16923 (KR); HAN, Jungsook, Anyang-si, Gyeonggi-do 14062 (KR)
(74) Representative: Schön, Christoph
(86) International application number: PCT/KR2023/021079
(87) International publication number: WO 2024/144075

(57) **Abstract**

The present application relates to an allulose syrup having an improved property.

## Description

### [TECHNICAL FIELD]

The present application relates to an allulose syrup having an improved property.

### [BACKGROUND ART]

Allulose is a rare saccharide having approximately 70% the sweetness of sucrose. As it is not metabolized upon ingestion, it has very low caloric content. It functions as a functional sweetener capable of preventing an increase in body fat and controlling blood glucose levels. Furthermore, although it exhibits a sweetness of 60-70% that of sucrose, allulose has almost no calories and possesses excellent solubility, which makes it suitable for application in a variety of food products.

During the manufacturing process of allulose, the allulose content in the product is low, thereby requiring purification and concentration steps. However, the application of concentrated syrups is limited, leading to a higher demand for crystalline or powdered forms. However, allulose has low crystallinity, making it difficult to crystallize. As a result, allulose is primarily provided in the form of allulose-rich syrup.

In addition, allulose syrup has a problem of easily browning, and therefore, there is a need to provide as allulose syrup that either prevents browning or exhibits improved color stability, thereby delaying or reducing the occurrence or browning.

### [DISCLOSURE]

### [TECHNICAL PROBLEM]

One embodiment of the present application provides a saccharide syrup composition, for example, an allulose syrup, comprising an allulose dimer crystal having a specific diffraction angle pattern, or a dissolved solution thereof.

An additional embodiment of the present application is to provide an agent for preventing browning of an allulose syrup or an agent of improving browning of an allulose syrup, for preventing, inhibiting, reducing, or improving browning of an allulose syrup, comprising an allulose dimer crystal having a specific diffraction angle pattern or a dissolved solution thereof.

Another embodiment of the present application relates to a method for preventing or improving browning of an allulose syrup, comprising a step of controlling a content of an allulose dimer crystal having a specific diffraction angle pattern or a dissolved solution thereof, in an allulose syrup.

Another embodiment of the present application relates to a method for reducing a yellowness change rate of an allulose syrup, comprising a step of controlling a content of an allulose dimer crystal having a specific diffraction angle pattern or a dissolved solution thereof, in an allulose syrup.

Another embodiment of the present application is to provide an agent of preventing moisture loss of an allulose syrup, for improving moisturizing property of an allulose syrup, comprising an allulose dimer crystal having a specific diffraction angle pattern or a dissolved solution thereof.

Another embodiment of the present application relates to a method for preventing moisture loss of an allulose syrup, comprising a step of controlling a content of an allulose dimer crystal having a specific diffraction angle pattern or a dissolved solution thereof, in an allulose syrup.

### [TECHNICAL SOLUTION]

An embodiment of the present application relates to a composition for preventing or improving browning of an allulose syrup, or a composition for preventing moisture loss, comprising an allulose dimer crystal having an X-ray powder diffraction pattern comprising peaks at three or more positions selected from 2-theta diffraction angles of 15.51±0.5°, 17.66±0.5°, 18.14±0.5°, 19.34±0.5°, 7.76±0.5° and 28.73±0.5° in X-ray powder diffraction (XRD) analysis, or a dissolved solution thereof.

Another embodiment of the present application relates to a saccharide syrup comprising allulose; and an allulose dimer crystal having an X-ray powder diffraction pattern comprising peaks at three or more positions selected from 2-theta diffraction angles of 15.51+0.5°, 17.66±0.5°, 18.14+0.5°, 19.34+0.5°, 7.76±0.5° and 28.73±0.5° in X-ray powder diffraction (XRD) analysis, or a dissolved solution thereof.

Hereinafter, the present application will be described in more detail.

The "allulose dimer" of the present application refers to a molecule in which two allulose molecules are chemically condensed. Specifically, the allulose dimer may be a substance having carbon 44.600 wt%, hydrogen 5.907 wt%, and oxygen 53.556 wt%, and may be an allulose dimer with a molecular formula of C₁₂H₂₀O₁₀ and a molecular weight of 324.11.

The allulose dimer of the present application may be a form of an allulose dimer crystal or a dissolved solution thereof. The allulose dimer crystal may have peaks at three or more positions selected from 2-theta diffraction angles of 15.51±0.5°, 17.66±0.5°, 18.14±0.5°, 19.34±0.5°, 7.76±0.5°, and 28.73±0.5° in the X-ray powder diffraction (XRD) analysis. Specifically, the allulose dimer crystal may have peaks at three or more positions selected from 2-theta diffraction angles of 15.51±0.2°, 17.66±0.2°, 18.14±0.2°, 19.34±0.2°, 7.76±0.2°, and 28.73±0.2° in the X-ray powder diffraction (XRD) analysis.

As one embodiment, the allulose dimer crystal may have an X-ray powder diffraction pattern comprising peaks at 2-theta diffraction angles of 15.51±0.5°, 17.66±0.5°, and 18.14±0.5°; 15.51±0.5°, 17.66±0.5°, 18.14±0.5°, and 19.34±0.5°; 15.51±0.5°, 17.66±0.5°, 18.14±0.5°, 19.34±0.5°, and 7.76±0.5°; or 15.51±0.5°, 17.66±0.5°, 18.14±0.5°, 19.34±0.5°, 7.76±0.5°, and 28.73±0.5° in the X-ray powder diffraction (XRD) analysis.

As one embodiment, the allulose dimer crystal may have an X-ray powder diffraction pattern comprising peaks at 2-theta diffraction angles of 15.51±0.2°, 17.66±0.2°, and 18.14±0.2°; 15.51+0.2°, 17.66+0.2°, 18.14+0.2°, and 19.34+0.2°; 15.51±0.2°, 17.66+0.2°, 18.14+0.2°, 19.34+0.2°, and 7.76+0.2°; or 15.51+0.2°, 17.66+0.2°, 18.14+0.2°, 19.34+0.2°, 7.76+0.2°, and 28.73+0.2° in the X-ray powder diffraction (XRD) analysis.

The peak may be a peak with a relative intensity of 30% or more, 35% or more, 40% or more, 45% or more, 50% or more, 55% or more, 60% or more, 65% or more, 70% or more, 75% or more, 80% or more, 85% or more, 90% or more, or 95% or more in the X-ray powder diffraction (XRD) analysis results. The relative intensity of the peak is expressed by the intensity of each peak as a relative numerical percent, based on 100% of the intensity of the peak having the maximum intensity.

Among the peaks, several peaks in order of highest relative intensity, or peaks with a relative intensity equal to or higher than a certain value, may be major peaks that define the crystalline form. For example, the major peaks may be 1 to 6, 1 to 5, 1 to 4, 1 to 3, or 1 to 2 peaks with the highest relative intensity. For example, the major peaks may be peaks with a relative intensity of 30% or more, 35% or more, 40% or more, 45% or more, 50% or more, 55% or more, 60% or more, 65% or more, 70% or more, 75% or more, 80% or more, 85% or more, 90% or more, or 95% or more.

Therefore, the diffraction angle of the allulose dimer crystal described above may be a diffraction angle at the major peak with a high relative intensity in the X-ray powder diffraction (XRD) analysis, and the order of description of the diffraction angles may be described from the diffraction angle with higher relative intensity of the peak in the X-ray powder diffraction (XRD) analysis.

As one embodiment, the allulose dimer crystal may have an X-ray powder diffraction pattern comprising peaks at 2-theta diffraction angles of 15.51±0.5°and 17.66±0.5°; 15.51±0.5°, 17.66+0.5° and 18.14+0.5°; 15.51+0.5°, 17.66+0.5°, 18.14+0.5° and 19.34+0.5°; 15.51+0.5°, 17.66+0.5°, 18.14+0.5°, 19.34+0.5° and 7.76+0.5°; or 15.51+0.5°, 17.66+0.5°, 18.14+0.5°, 19.34+0.5°, 7.76+0.5° and 28.73+0.5°, in order of relative intensity of the peak from high to low in the X-ray powder diffraction (XRD) analysis. Specifically, the allulose dimer crystal may have an X-ray powder diffraction pattern comprising peaks at 2-theta diffraction angles of 15.51±0.2°and 17.66±0.2°; 15.51±0.2°, 17.66±0.2° and 18.14±0.2°; 15.51±0.2°, 17.66±0.2°, 18.14±0.2° and 19.34±0.2°; 15.51±0.2°, 17.66±0.2°, 18.14±0.2°, 19.34±0.2° and 7.76±0.2°; or 15.51±0.2°, 17.66±0.2°, 18.14±0.2°, 19.34±0.2°, 7.76±0.2° and 28.73±0.2°, in order of relative intensity of the peak from high to low in the X-ray powder diffraction (XRD) analysis.

As one embodiment, the allulose dimer crystal may have an X-ray powder diffraction pattern in which the peak with the highest relative intensity is positioned at a 2-theta diffraction angle of 15.51±0.5° or 15.51±0.2° in the X-ray powder diffraction (XRD) analysis.

As one embodiment, the allulose dimer crystal may have an X-ray powder diffraction pattern in which 2 peaks with the highest relative intensity are positioned at 2-theta diffraction angles of 15.51±0.5° and 17.66±0.5°; or 15.51±0.2° and 17.66±0.2° in the X-ray powder diffraction (XRD) analysis.

As one embodiment, the allulose dimer crystal may have an X-ray powder diffraction pattern in which 3 peaks with the highest relative intensity are positioned at 2-theta diffraction angles of 15.51±0.5°, 17.66±0.5° and 18.14±0.5°; or 15.51±0.2°, 17.66±0.2° and 18.14±0.2° in the X-ray powder diffraction (XRD) analysis.

The allulose dimer crystals may have a melting point of 210±5°C, 210±3°C, 210±2°C, 210±1°C, 210.5±5°C, 210.5±3°C, 210.5±2°C, 210.5±1°C, 210.5±0.5°C, 210.7±5°C, 210.7±3°C, 210.7±2°C, 210.7±1°C, or 210.7±0.5°C.

The allulose dimer crystal may have a solubility to water of 5 to 20 g/100g, 5 to 15 g/100g, 5 to 12 g/100g, 5 to 10 g/100g, 7 to 20 g/100g, 7 to 15 g/100g, 7 to 12 g/100g, 7 to 10 g/100g, 8 to 20 g/100g, 8 to 15 g/100g, 8 to 12 g/100g, 8 to 10 g/100g, 9 to 20 g/100g, 9 to 15 g/100g, 9 to 12 g/100g, or 9 to 10 g/100g.

The allulose dimer may have a retention time of 11 to 13 minutes analyzed by a gas chromatography (GC) analysis method.

One embodiment of the present application relates to a composition for preventing or improving browning of an allulose syrup, comprising the allulose dimer crystal or a dissolved solution thereof. The allulose dimer crystal or dissolved solution thereof may be comprised in the allulose syrup, thereby preventing or improving browning of the allulose syrup. Accordingly, another embodiment of the present application relates to an allulose syrup in which browning is prevented or improved, comprising the allulose dimer crystal or dissolved solution thereof.

In the present application, "preventing browning" may be used as a meaning of comprising inhibiting or delaying occurrence of browning. The preventing browning, inhibiting browning, and delaying browning and the like refer to that the increase rate of the level of browning (for example, yellowness numerical value) compared to immediately after preparation is lower than the control group.

In the present application, "improving browning" may be used as a meaning of comprising reducing the degree of browning, for example, reducing yellowness. The improving browning may mean that the level of browning (for example, yellowness numerical value) compared to immediately after preparation is lowered.

The browning may be for example, browning due to one or more selected from the group consisting of heat, pH and storage time. The browning comprises non-enzymatic browning reactions including amino carbonyl reaction, melanoidine reaction, Maillard reaction and the like that occur when an amino group and a carbonyl group coexist, and this may include browning resulting from the formation of osones, browning caused by the formation of intermediates such as unsaturated 3,4 dieoxyosone, browning that a furfural-generating cyclic substance comprising HMF (hydroxymethyl furfural) becomes melanoidine due to a reaction with an amino acid and has brown color, and a browning process of brown pigments and the like derived from the decomposition products of saccharide.

Another embodiment of the present application relates to a composition for preventing moisture loss of an allulose syrup, comprising the allulose dimer. The allulose dimer crystal or dissolved solution thereof may be comprised in an allulose syrup and prevent moisture loss of the allulose syrup. Accordingly, the other embodiment of the present application relates to an allulose syrup in which moisture loss is prevented, comprising the allulose dimer crystal or dissolved solution thereof.

In the present application, "preventing moisture loss" may be used as a meaning of including inhibiting or delaying loss of moisture comprised in a saccharide syrup, for example, an allulose syrup. The preventing moisture loss means that the amount of moisture loss compared to immediately after preparation is lower than the control group.

The allulose dimer crystal or dissolved solution thereof according to one embodiment of the present application may be comprised in an amount of 0.025wt% or more, 0.03wt% or more, 0.035wt% or more, 0.04wt% or more, 0.045wt% or more, 0.05wt% or more, 0.055wt% or more, 0.06wt% or more, 0.065wt% or more, 0.07wt% or more, 0.075wt% or more, 0.08wt% or more, 0.085wt% or more, 0.09wt% or more, 0.095wt% or more, 0.1wt% or more, 0.2wt% or more, 0.3wt% or more, 0.4wt% or more, 0.5wt% or more, 0.6wt% or more, 0.7wt% or more, 0.8wt% or more, 0.9wt% or more, 1wt% or more, 1.5wt% or more, 2wt% or more, 2.5wt% or more, 3wt% or more, 3.5wt% or more, 4wt% or more, 4.5wt% or more, or 5wt% or more, based on 100wt% of the solid content of the allulose syrup. The content of the allulose dimer crystal or dissolved solution thereof may be measured based on the gas chromatography (GC) analysis method.

The content of the allulose dimer crystal or dissolved solution thereof may be 20wt% or less, 19wt% or less, 18wt% or less, 17wt% or less, 16wt% or less, 15wt% or less, 14wt% or less, 13wt% or less, 12wt% or less, 11wt% or less, or 10wt% or less, based on 100wt% of the solid content of the allulose syrup.

Other embodiment of the present application relates to a saccharide syrup, comprising an allulose; and an allulose dimer crystal having an X-ray powder diffraction pattern comprising peaks at three or more positions selected from 2-theta diffraction angles of 15.51±0.5°, 17.66±0.5°, 18.14±0.5°, 19.34±0.5°, 7.76±0.5° and 28.73±0.5° in X-ray powder diffraction (XRD) analysis, or a dissolved solution thereof. The allulose dimer crystal or dissolved solution thereof is as described above.

The solid content of the saccharide syrup may be 20 to 90wt%, 20 to 85wt%, 20 to 80wt%, 20 to 75wt%, 20 to 70wt%, 25 to 90wt%, 25 to 85wt%, 25 to 80wt%, 25 to 75wt%, 25 to 70wt%, 30 to 90wt%, 30 to 85wt%, 30 to 80wt%, 30 to 75wt%, 30 to 70wt%, 40 to 90wt%, 40 to 85wt%, 40 to 80wt%, 40 to 75wt%, 40 to 70wt%, 50 to 90wt%, 50 to 85wt%, 50 to 80wt%, 50 to 75wt%, 50 to 70wt%, 60 to 90wt%, 60 to 85wt%, 60 to 80wt%, 60 to 75wt%, or 60 to 70wt%.

The saccharide syrup may comprise the allulose dimer or dissolved solution thereof in an amount of 0.025wt% or more, 0.03wt% or more, 0.035wt% or more, 0.04wt% or more, 0.045wt% or more, 0.05wt% or more, 0.055wt% or more, 0.06wt% or more, 0.065wt% or more, 0.07wt% or more, 0.075wt% or more, 0.08wt% or more, 0.085wt% or more, 0.09wt% or more, 0.095wt% or more, 0.1wt% or more, 0.2wt% or more, 0.3wt% or more, 0.4wt% or more, 0.5wt% or more, 0.6wt% or more, 0.7wt% or more, 0.8wt% or more, 0.9wt% or more, 1wt% or more, 1.5wt% or more, 2wt% or more, 2.5wt% or more, 3wt% or more, 3.5wt% or more, 4wt% or more, 4.5wt% or more, or 5wt% or more, based on 100wt% of the solid content. The content of the allulose dimer crystal or dissolved solution thereof may be measured by the gas chromatography (GC) analysis method.

The saccharide syrup may comprise the allulose dimer or dissolved solution thereof in an amount of 20wt% or less, 19wt% or less, 18wt% or less, 17wt% or less, 16wt% or less, 15wt% or less, 14wt% or less, 13wt% or less, 12wt% or less, 11wt% or less, or 10wt% or less, based on 100wt% of the solid content.

The saccharide syrup may be prevented from browning, and the preventing browning is as described above. In the Examples of the present application, it was confirmed that the increase in yellowness of the saccharide syrup composition comprising an allulose dimer or a solution thereof was suppressed compared to the control.

Specifically, a yellowness change rate after storing the saccharide syrup according to one embodiment of the present application at a temperature of 60°C for 48 hours, may be 70% or less, 65% or less, 60% or less, 55% or less, 50% or less, 45% or less, 40% or less, 35% or less, 30% or less, 25% or less, 20% or less, 15% or less, 10% or less, 5% or less, 4% or less, 3% or less, 2% or less, or 1% or less, based on 100% of the yellowness measured immediately after preparation.

Specifically, a yellowness change rate after storing the saccharide syrup according to one embodiment of the present application at a temperature of 60°C for 3 days, may be 260% or less, 250% or less, 240% or less, 230% or less, 200% or less, 180% or less, 150% or less, or 130% or less based on 100% of the yellowness measured immediately after preparation.

Specifically, a yellowness change rate after storing the saccharide syrup according to one embodiment of the present application at a temperature of 60°C for 6 days may be 1400% or less, 1350% or less, 1300% or less, 1200% or less, or 1100% or less, based on 100% of the yellowness measured immediately after preparation.

In one embodiment of the present application, the saccharide syrup may have improved browning, and the improving browning is as described above. In the Example of the present application, it was confirmed that the yellowness of the saccharide syrup comprising an allulose dimer or a solution thereof according to an embodiment of the present application compared to the initial value.

Specifically, a yellowness change rate after storing the saccharide syrup according to one embodiment of the present application at a temperature of 60°C for 48 hours may be -5% or less, -10% or less, -15% or less, -18% or less, -20% or less, -25% or less, or -30% or less, based on 100% of the yellowness measured immediately after preparation.

The yellowness change rate may be calculated by [(measured yellowness)-(yellowness immediately after preparation)]/(yellowness immediately after preparation) X 100(%).

As one example, the saccharide syrup according to one embodiment of the present application may have a yellowness change rate after storage at a temperature of 60°C for 48 hours of -50 to 70%, -50 to 65%, -50 to 60%, -50 to 55%, -50 to 50%, -50 to 45%, -50 to 40%, - 40 to 70%, -40 to 65%, -40 to 60%, -40 to 55%, -40 to 50%, -40 to 45%, -40 to 40%, -35 to 70%, -35 to 65%, -35 to 60%, -35 to 55%, -35 to 50%, -35 to 45%, or -35 to 40%, based on 100% of the yellowness measured immediately after preparation.

The saccharide syrup may be prevented from moisture loss, and the preventing moisture loss is as described above. In the Example of the present application, it was confirmed that the saccharide syrup comprising the allulose dimer or dissolved solution thereof according to one embodiment of the present application exhibited less moisture loss compared to the control group.

Specifically, a moisture content after storing the saccharide syrup according to one embodiment of the present application at 60°C for 1 day may be 57wt% or more, 58wt% or more, 59wt% or more, 60wt% or more, 61wt% or more, 62wt% or more, 63wt% or more, 64wt% or more, 65wt% or more, 66wt% or more, 67wt% or more, 68wt% or more, 69wt% or more, or 70wt% or more, based on 100wt% of the moisture content immediately after preparation.

The saccharide syrup may further comprise at least one selected from the group consisting of monosaccharides other than allulose, disaccharides, sugar alcohols, oligosaccharides, high-intensity sweeteners, dietary fibers, proteins, vitamins, flavorings and colorants.

The monosaccharides may be one or more selected from the group consisting of fructose, glucose and galactose.

The disaccharides may be one or more selected from the group consisting of sucrose, lactose and maltose.

The sugar alcohols may be one or more selected from the group consisting of xylitol, erythritol, sorbitol, maltitol, mannitol, isomalt, and lactitol, but not limited thereto.

The high-intensity sweeteners may be one or more selected from the group consisting of stevioglucoside, stevia extract, aspartic acid, aspartame, stevioside, enzymatically modified stevia, sucralose, saccharin, rebaudioside, monk fruit, mogroside, acesulfame potassium, thaumatin, brazzein, monellin, miraculin, and pentadin, but not limited thereto.

The dietary fibers may include water-soluble dietary fibers and/or insoluble dietary fibers, and the water-soluble dietary fibers may be one or more selected from the group consisting of arabinoxylan, fructan, inulin, pectin, alginate, agar, raffinose, polydextrose, maltodextrin, and indigestible maltodextrin, and the insoluble dietary fibers may be one or more selected from the group consisting of fibrin (cellulose), chitin, hemicellulose, lignin, xanthan gum, and resistant starch, but not limited thereto.

The proteins may include animal proteins and/or vegetable proteins, but not limited thereto.

The vitamins may include fat-soluble vitamins and/or water-soluble vitamins, but not limited thereto.

There is no particular limitation on the type of the flavorings as long as they are edible flavorings, and for example, they may be one or more selected from strawberry flavor, vanilla flavor, chocolate flavor, green tea flavor, apple flavor, grape flavor, blueberry flavor, raspberry flavor, cranberry flavor, watermelon flavor, orange flavor, banana flavor, caramel flavor, pistachio flavor, milk flavor, yogurt flavor, lemon flavor, chestnut flavor, peanut flavor, coffee flavor, black tea flavor, melon flavor, grain flavor, sweet potato flavor, pear flavor, red bean flavor, walnut flavor, almond flavor, peach flavor, cherry flavor, Hotteok flavor, tomato flavor, soda flavor, coke flavor, pumpkin flavor, and corn flavor, and the like.

There is no particular limitation on the type of colorants as long as they are edible colorants, and for example, they may be one or more colorants selected from the group consisting of blue colorants such as Gardenia blue colorant and spirulina colorant, yellow colorants such as Gardenia yellow colorant, turmeric colorant, annatto colorant, and carotene, red colorants such as beet colorant, cochineal colorant, lac colorant, Monascus red colorant, anthocyanin, and paprika extract colorant, violet colorants such as red cabbage colorant, purple sweet potato colorant, and grape skin extract colorant, brown colorants such as cacao colorant and caramel colorant, green colorants such as green spirulina, and chlorella colorant, and the like. Various colorants may be made by combining various kinds of colorants as above.

Other embodiment of the present application relates to a method for preparation of a saccharide syrup comprising allulose, comprising a step of controlling a content of an allulose dimer crystal having an X-ray powder diffraction pattern comprising peaks at three or more positions selected from 2-theta diffraction angles of 15.51±0.5°, 17.66±0.5°, 18.14±0.5°, 19.34±0.5°, 7.76±0.5°, and 28.73±0.5° in the X-ray powder diffraction (XRD) analysis or a dissolved solution thereof, in a saccharide syrup comprising allulose.

Other embodiment of the present application relates to a method for preventing or improving browning of a saccharide syrup comprising allulose, comprising a step of controlling a content of an allulose dimer crystal having an X-ray powder diffraction pattern comprising peaks at three or more positions among 2-theta diffraction angles of 15.51±0.5°, 17.66±0.5°, 18.14±0.5°, 19.34±0.5°, 7.76±0.5°, and 28.73±0.5° in the X-ray powder diffraction (XRD) analysis or a dissolved solution thereof.

Other embodiment of the present application relates to a method for preventing moisture loss of a saccharide syrup comprising allulose, comprising a step of controlling a content of an allulose dimer crystal having an X-ray powder diffraction pattern comprising peaks at three or more positions among 2-theta diffraction angles of 15.51±0.5°, 17.66±0.5°, 18.14±0.5°, 19.34±0.5°, 7.76±0.5°, and 28.73±0.5° in the X-ray powder diffraction (XRD) analysis or a dissolved solution thereof.

The step of controlling, may be controlling the content of the allulose dimer crystal or dissolved solution thereof to 0.025wt% or more, 0.03wt% or more, 0.035wt% or more, 0.04wt% or more, 0.045wt% or more, 0.05wt% or more, 0.055wt% or more, 0.06wt% or more, 0.065wt% or more, 0.07wt% or more, 0.075wt% or more, 0.08wt% or more, 0.085wt% or more, 0.09wt% or more, 0.095wt% or more, 0.1wt% or more, 0.2wt% or more, 0.3wt% or more, 0.4wt% or more, 0.5wt% or more, 0.6wt% or more, 0.7wt% or more, 0.8wt% or more, 0.9wt% or more, 1wt% or more, 1.5wt% or more, 2wt% or more, 2.5wt% or more, 3wt% or more, 3.5wt% or more, 4wt% or more, 4.5wt% or more, or 5wt% or more, based on 100wt% of the solid content of the saccharide syrup. The content of the dissolved solution may be the content of the allulose dimer crystal excluding a solvent.

The step of controlling may comprise for example, a step of adding the allulose dimer crystal or dissolved solution thereof into the saccharide syrup, or a step of increasing the content of the allulose dimer crystal or dissolved solution thereof comprised in the saccharide syrup.

### [ADVANTAGEOUS EFFECTS]

The composition for preventing or improving browning, composition for preventing moisture loss, or saccharide syrup according to one embodiment of the present application, comprises the allulose dimer crystal according to one embodiment of the present application or dissolved solution thereof, and therefore, it can prevent or reduce browning of the allulose syrup that occurs over storage time, and can minimize loss of moisture comprised in the allulose syrup.

### [BRIEF DESCRIPTION OF THE DRAWINGS]

FIG. 1 is a graph showing the solubility of the allulose dimer crystal according to one embodiment of the present application.
FIG. 2 is a drawing showing the gas chromatography (GC) analysis result of the allulose dimer crystal according to one embodiment of the present application.
FIG. 3 is a drawing showing the yellowness change of the saccharide syrup according to one embodiment of the present application.
FIG. 4 is a drawing showing the yellowness change of the saccharide syrup according to one embodiment of the present application by subdividing the allulose dimer crystal content.
FIG. 5 is a drawing showing the yellowness change when storing the saccharide syrup according to one embodiment of the present application for a long period of time.

### [MODE FOR INVENTION]

Hereinafter, the present application will be described in more detail by the following Examples. However, these Examples are intended to illustrate the present application only, but the scope of the present application is not limited by these Examples.

### Example 1: Separation of Allulose Dimer Crystals

Allulose syrup with allulose purity of 95 wt% or more and the solid content of 68~72 wt% was titrated to pH 3.5 or less and then stored at 35°C until the allulose content decreased to 80 wt% or less.

When the allulose content decreased to 80wt% or less, the allulose syrup was refrigerated to promote crystallization of allulose dimers. After that, when the formation of allulose dimer crystals was visually confirmed, the allulose dimer crystals were separated from the syrup using ethanol. The separated allulose dimer crystals were dried in a dry oven to evaporate ethanol, and then peak elution was monitored by HPLC and fractions were collected based on detector signals or retention time to separate purified products. Specifically, for HPLC was performed using a refractive index (RI) detector with a Biorad HPX-87C column maintained at 80°C, and the mobile phase was distilled water (DW) at a flow rate of 0.6 mL/min to separate and analyze the allulose dimer. Through this, pure allulose dimers (purity 99wt%) were obtained and then used for subsequent experiments.

### Example 2: Element Analysis of Allulose Dimer Crystals

For substance analysis of the allulose dimer crystals obtained in Example 1, element analysis was performed using an elemental analyzer. The analysis temperature of 1,150°C was set using 1.8 mg of the sample, and sulfanilic acid and benzoic acid were used as standard substances. As a result of the analysis, it was confirmed as a substance having C 44.600, H 5.907, O 53.556 wt%, and the molecular formula was confirmed to be C₁₂H₂₀O₁₀ and the molecular weight was confirmed to be 324.11.

### Example 3: Mass Spectrometry Analysis of Allulose Dimer Crystals

For mass spectrometry of the allulose dimer crystals obtained in Example 1, analysis was performed using a Hing Resolution Mass Spectrometer. The ionization conditions for the analysis were analyzed with ion mode FAB (Fast Atom Bombardment).

As a result of the mass spectrometry, the molecular weight of the allulose dimer was confirmed to be 324.11, which was consistent with the analysis result of Example 2.

### Example 4: X-ray Diffraction Analysis (XRD)

For the allulose dimer crystals obtained in Example 1, X-ray diffraction analysis was performed according to the following specific analysis conditions, and top (relative intensity %) 6 peaks were selected from the X-ray diffraction analysis result of the allulose dimer crystals and shown in Table 1.

**[Table 1]**

| Angle 2-Theta degree | Relative Intensity (%) |
|---|---|
| 15.51° | 100.0 |
| 17.66° | 73.7 |
| 18.14° | 60.17 |
| 19.34° | 48.28 |
| 7.76° | 41.68 |
| 28.73° | 38.12 |

As shown in Table 1, the allulose dimer crystals obtained in Example 1 exhibited the 2-theta values of the top 3 peaks of 15.51°, 17.66° and 18.14°, the 2-theta values of the top 4 peaks of 15.51°, 17.66°, 18.14° and 19.34°, the 2-theta values of the top 5 peaks of 15.51°, 17.66°, 18.14°, 19.34° and 7.76°, and the 2-theta values of the top 6 peaks of 15.51°, 17.66°, 18.14°, 19.34°, 7.76° and 28.73° in the powder X-ray spectroscopy spectrum.

### Example 5: Melting Point Analysis of Allulose Dimer Crystals (DSC Analysis)

For the allulose dimer crystals obtained in Example 1, the melting point was confirmed using differential scanning calorimetry. The analysis temperature range was -10°C ~ 250°C, and 4 mg of the allulose dimer crystal sample was quantified and analyzed.

As a result of the melting point analysis, the melting point of the allulose dimer crystals was confirmed to be about 210.7°C.

### Example 6: Solubility Analysis of Allulose Dimer Crystals

To analyze the solubility of the allulose dimer crystals obtained in Example 1, the solubility was evaluated at a temperature of 25°C to 80°C using water as a solvent.

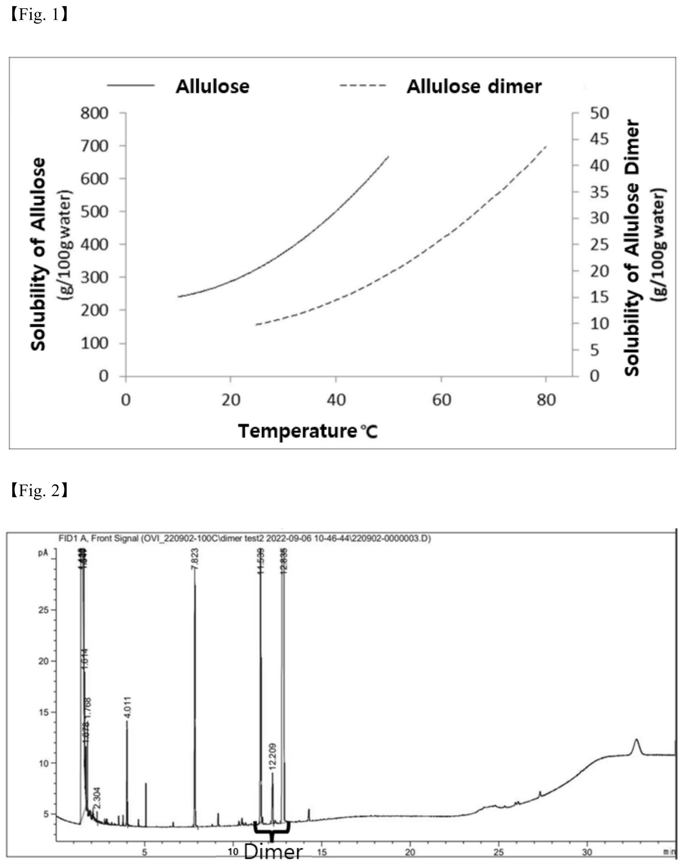

As a result of the analysis of solubility to water, 34g/100g (water) of allulose was dissolved based on the solvent temperature of 25°C, whereas the allulose dimer crystals were confirmed to be 9.2g/100g (water). The graph of the solubility of the allulose and allulose dimer crystals was shown in FIG. 1.

### Example 7: GC Analysis of Allulose Dimer Crystals

100mg to 300mg of the allulose dimer crystal sample obtained in Example 1 was titrated, and 100ml pyridine mixed with phenyl beta-D-glucopyranoside of 30 mg was taken by 10ml, and mixed with the allulose dimer crystal samples. 0.5 ml of the mixed solution was taken and placed in a 2ml to 4ml reagent bottle, and evaporated and dried under s nitrogen stream, and then 0.5 ml of a solution in which hydroxylamine hydrochloride of 4g was mixed to pyridine of 100ml was taken and mixed to the evaporated and dried sample, and then stirred sufficiently to dissolve the dried materials inside, and then left at 70°C for 40 minutes. The reactants were left at a room temperature after 40 minutes, and 0.4ml BSTFA and 0.1ml N-trimethylsilylimidazole (TSIM) were added. The stopper was closed again, and heated at 70°C for 30 minutes, and then cooled at a room temperature, and then used as a sample for GC analysis. The GC analysis conditions were as Table 2, and the GC analysis dimer content analysis formula is as follows: Dimer (%) = (GC peak 11~13min peak surface area of substance/Surface area of internal standard substance peak) * (Weight of internal standard substance added during sample pretreatment/Sample weight) * (100/Dimer reaction coefficient)
- Dimer reaction coefficient: 1.15

**[Table 2]**

| | |
|---|---|
| Analysis equipment | Agilent company 5977C GC/MSD |
| Detector | FID (Flame ionization detector) |
| Analysis column | DB1 capillary tube 30 meters (J&W scientific ref.:123-1032) |
| Column temperature | Increasing from 200°C to 280°C at a rate of 5°C/min, and then maintaining at 280°C for 6 minutes, and then increasing to 320°C at 5°C/min again, and then maintaining at 320°C for 5 minutes |
| Injector temperature | 300°C |
| Detector temperature | 300°C |
| Pressure | 14 psi |
| Vector gas | Helium |
| Injection mode | Division (division flow: 80ml/min) |
| Injected volume | 1.2ul |

The GC analysis result of the allulose dimer crystals was shown in FIG. 2. As a result of the GC analysis, it was confirmed that peaks were detected at 11 to 13 minutes.

### Example 8: Anti-browning Effect of Allulose Dimers (1)

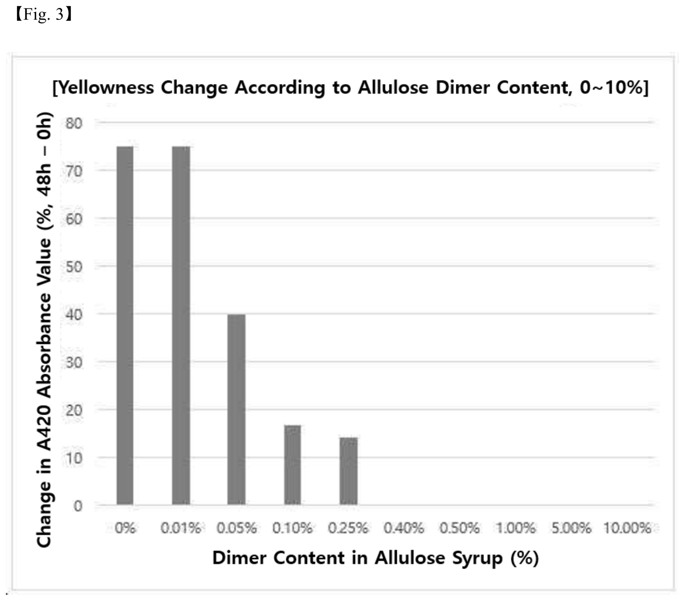

The effect of the allulose dimers obtained in Example 1 on browning of allulose syrup was confirmed. Allulose crystals and allulose dimer crystals with the purity of 99.99% were mixed in a weight ratio according to Table 3 to prepare liquid samples, and the solid content was adjusted to 68 wt%. Each sample was stored at a temperature of 60°C and sampled at 0hr and 48hr after storage, and the yellowness was analyzed at 420nm in a spectrophotometer with a solid content of 30 wt%, and the yellowness change rate (%) at 48h compared to 0hr was calculated as the following Equation 1, and shown in Table 4 and FIG. 3: Yellowness change rate (%) = [(48h yellowness)-(0h yellowness)]/(0h yellowness) × 100%

**[Table 3]**

| Allulose (wt%) | Allulose dimer (wt%) |
|---|---|
| 100.0 | 0 |
| 99.99 | 0.01 |
| 99.95 | 0.05 |
| 99.90 | 0.10 |
| 99.75 | 0.25 |
| 99.60 | 0.40 |
| 99.50 | 0.50 |
| 99.00 | 1.00 |
| 95.00 | 5.00 |
| 90.00 | 10.00 |

**[Table 4]**

| Allulose dimer (wt%) | Yellowness change rate (%) |
|---|---|
| 0 | 75 |
| 0.01 | 75 |
| 0.05 | 40 |
| 0.10 | 16.67 |
| 0.25 | 14.29 |
| 0.40 | 0.00 |
| 0.50 | 0.00 |
| 1.00 | -12.50 |
| 5.00 | -26.23 |
| 10.00 | -30.12 |

As shown in Table 4 and FIG. 3 above, as a result of comparing the progress of browning depending on the allulose dimer content according to one embodiment of the present application, it could be confirmed that the rate of browning was reduced from the sample with the allulose dimer content of 0.05 wt% or more compared to the non-added control group, and it could be seen that there was no change in yellowness or it was rather decreased from the sample with the allulose dimer content of 0.4 wt% or more compared to 0 hour. Therefore, the allulose syrup comprising the allulose dimer crystals according to one embodiment of the present application or dissolved solution thereof at a certain concentration or more had an effect of delaying browning.

### Example 9: Anti-browning Effect of Allulose Dimers (2)

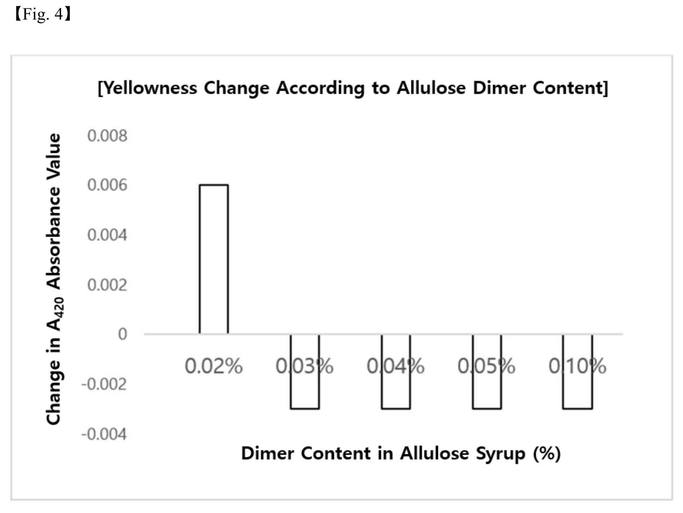

In order to confirm the anti-browning effect according to the allulose dimer crystal content in more detail, the allulose dimer crystal content was divided into 0.01wt% units as the weigh ratio described in Table 5, and allulose syrup was prepared by the same method as Example 8, and stored at 60°C for 48 hours by the same method as Example 8 to accelerate browning. After 48 hours, the allulose syrup was adjusted to a concentration of 68wt%, and the yellowness was analyzed at 420nm using a spectrophotometer, and the yellowness change rate (%) at 48hr compared to 0hr was calculated as the Equation 1 and shown in Table 6 and FIG. 4.

**[Table 5]**

| Allulose (wt%) | Allulose dimer (wt%) |
|---|---|
| 99.98 | 0.02 |
| 99.97 | 0.03 |
| 99.96 | 0.04 |
| 99.95 | 0.05 |
| 99.90 | 0.10 |

**[Table 6]**

| Allulose dimer (wt%) | Yellowness change rate (%) |
|---|---|
| 0.02 | 85.71 |
| 0.03 | -18.75 |
| 0.04 | -18.75 |
| 0.05 | -18.75 |
| 0.10 | -18.75 |

As shown in Table 6 and FIG. 4, browning was prevented in the allulose syrup comprising 0.03 wt% or more of allulose dimers, and in particular, the reduction in yellowness compared to the control group was also observed.

### Example 10: Anti-browning Effect of Allulose Dimers During Long-Term Storage

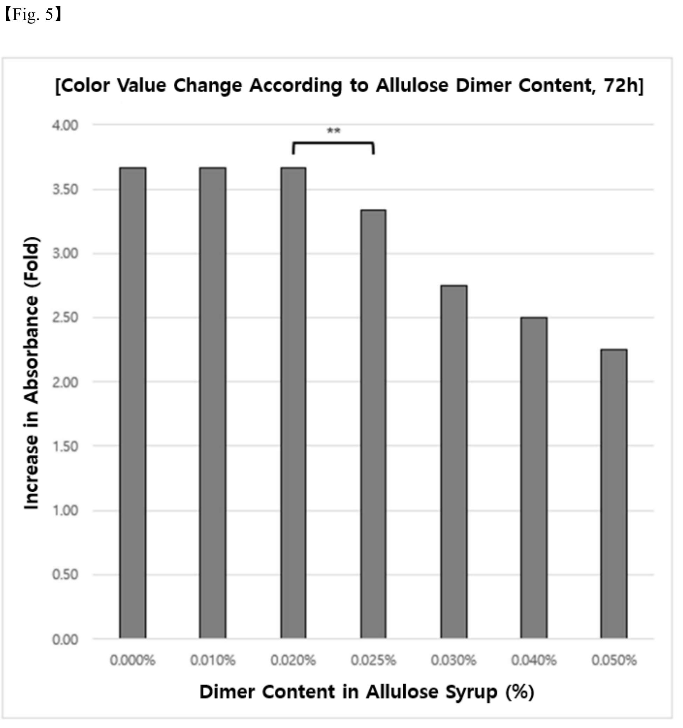

In order to confirm the effect of delaying browning according to the allulose dimer content during long-term storage of saccharide syrup, the allulose syrup with a solid content of 71wt% was prepared by the same method as Example 8 as the weight ratio described in Table 7, and stored at 60°C for 6 days for a long period of time to accelerate browning. After 3 days and 6 days of storage, the allulose syrup was adjusted to a concentration of 30 wt% and then the color value was analyzed at 420nm using a spectrophotometer, and the yellowness change rate (%) on Day 3 of storage and Day 6 of storage compared to Day 0 of storage was calculated and shown in Table 8 and FIG. 5.

**[Table 7]**

| Allulose (wt%) | Allulose dimer (wt%) |
|---|---|
| 100.0 | 0.00 |
| 99.99 | 0.01 |
| 99.98 | 0.02 |
| 99.975 | 0.025 |
| 99.97 | 0.03 |
| 99.96 | 0.04 |
| 99.95 | 0.05 |

**[Table 8]**

| Allulose dimer (wt%) | Yellowness change rate (%) (Day 3) | Yellowness change rate (%) (Day 6) |
|---|---|---|
| 0 | 266.67 | 1466.667 |
| 0.01 | 266.67 | 1466.667 |
| 0.02 | 266.67 | 1433.333 |
| 0.025 | 233.33** | 1366.667* |
| 0.03 | 175.00** | 1075** |
| 0.04 | 150.00** | 1025** |
| 0.05 | 125.00** | 1025** |

| | | |
|---|---|---|
| * p<0.05, ** p<0.005 | | |

As shown in Table 7 and FIG. 5, it could be confirmed that browning was prevented in the allulose syrup comprising 0.025 wt% or more of allulose dimers.

### Example 11: Moisture Loss Prevention Effect of Allulose Dimers

In order to confirm the effect of preventing moisture loss according to the allulose dimer content when saccharide syrup was stored for a long period of time, the allulose syrup with the solid content of 71wt% was prepared by mixing at a weight ratio described in Table 9 as the same method as Example 8, and 10g was titrated in a dish and stored at 60°C for 1 day to induce moisture evaporation. After storing, the moisture content evaporated from the allulose syrup was analyzed using reduced-pressure drying, and the moisture loss rate (%) inside the saccharide syrup on Day 1 of storage compared to Day 0 of storage was calculated and shown in Table 10.

**[Table 9]**

| Allulose (wt%) | Allulose dimer (wt%) |
|---|---|
| 100.0 | 0.0 |
| 99.5 | 0.5 |
| 99.0 | 1.0 |
| 98.0 | 2.0 |
| 97.0 | 3.0 |
| 96.0 | 4.0 |
| 95.0 | 5.0 |

**[Table 10]**

| Allulose dimer (wt%) | Moisture content (%) (0h) | Moisture content (%) (6h) | Moisture content (%) (12h) | Moisture content (%) (24h) |
|---|---|---|---|---|
| 0.0 | 100 | 85.1 | 65.4 | 56.7 |
| 0.5 | 100 | 86.4 | 67.7 | 57.9 |
| 1.0 | 100 | 89.2** | 71.6** | 61.3** |
| 2.0 | 100 | 91.6** | 74.5** | 63.9** |
| 3.0 | 100 | 93.7** | 77.0** | 66.1** |
| 4.0 | 100 | 95.6** | 80.6** | 68.7** |
| 5.0 | 100 | 96.2** | 84.5** | 72.4** |

| | | | | |
|---|---|---|---|---|
| * p<0.05, ** p<0.005 | | | | |

As shown in Table 10, as a result of measuring moisture loss based on 100% of the initial moisture content, it was confirmed that the higher the allulose dimer content, the more moisture loss was delayed. In particular, it could be found that the moisture loss was significantly prevented at 1.0wt% or more of the allulose dimer content.

## Claims

1. A composition comprising an allulose dimer crystal having an X-ray powder diffraction pattern comprising peaks at three or more positions selected from 2-theta diffraction angles of 15.51±0.5°, 17.66±0.5°, 18.14±0.5°, 19.34±0.5°, 7.76±0.5° and 28.73±0.5° in X-ray powder diffraction (XRD) analysis, or a dissolved solution thereof.

2. The composition according to claim 1, wherein the allulose dimer crystal has an X-ray powder diffraction pattern comprising peaks at positions of 2-theta diffraction angles of 15.51±0.5°, 17.66±0.5° and 18.14±0.5° in X-ray powder diffraction (XRD) analysis.

3. The composition according to claim 1, wherein the allulose dimer crystal has an X-ray powder diffraction pattern comprising peaks at positions of 2-theta diffraction angles of 15.51±0.5°, 17.66±0.5°, 18.14±0.5°, and 19.34±0.5° in X-ray powder diffraction (XRD) analysis.

4. The composition according to claim 1, wherein the allulose dimer crystal has an X-ray powder diffraction pattern comprising peaks at positions of 2-theta diffraction angles of 15.51±0.5°, 17.66±0.5°, 18.14±0.5°, 19.34±0.5° and 7.76±0.5° in X-ray powder diffraction (XRD) analysis.

5. The composition according to claim 1, wherein the allulose dimer crystal has an X-ray powder diffraction pattern comprising peaks at positions of 2-theta diffraction angles of 15.51±0.5°, 17.66±0.5°, 18.14±0.5°, 19.34±0.5°, 7.76±0.5°, and 28.73±0.5° in X-ray powder diffraction (XRD) analysis.

6. The composition according to claim 1, wherein the allulose dimer crystal has a melting point of 210±5°C.

7. The composition according to claim 1, wherein the allulose dimer crystal has a solubility to water of 5 to 20 g/100g.

8. The composition according to claim 1, wherein a molecular formula of the allulose dimer crystal is C₁₂H₂₀O₁₀.

9. The composition according to claim 1, wherein a molecular weight of the allulose dimer crystal is 324.11.

10. The composition according to claim 1, wherein the allulose dimer has a retention time of 11 to 13 minutes analyzed by a gas chromatography (GC) analysis method.

11. The composition according to any one claim of claims 1 to 10, wherein the composition is for preventing or improving browning of an allulose syrup.

12. The composition according to claim 11, wherein the preventing or improving browning is **characterized by** that a yellowness change rate of -50% to 70%, when an allulose syrup is stored under the condition of 60°C for 48 hours.

13. The composition according to claim 11, wherein the browning is caused by at least one selected from the group consisting of heat, pH, and storage time.

14. The composition according to claim 11, wherein the allulose dimer is comprised by 0.025wt% or more, based on 100wt% of the solid content.

15. The composition according to claim 11, wherein the allulose dimer is comprised by 0.025 wt% or more, based on a gas chromatography (GC) analysis method.

16. The composition according to any one claim of claims 1 to 10, wherein the composition is for preventing moisture loss of an allulose syrup.

17. The composition according to claim 16, wherein the preventing moisture loss is **characterized by** that a moisture content after storing an allulose syrup under the condition of 60°C for 24 hours is 57 wt% or more based on 100wt% of the moisture content immediately after preparation.

18. The composition according to claim 16, wherein the allulose dimer is comprised by 1wt% or more, based on 100wt% of the solid content.

19. The composition according to claim 16, wherein the allulose dimer is comprised by 1wt% or more, based on a gas chromatography (GC) analysis method.

20. A saccharide syrup comprising
an allulose; and
an allulose dimer crystal having an X-ray powder diffraction pattern comprising peaks at three or more positions selected from 2-theta diffraction angles of 15.51±0.5°, 17.66±0.5°, 18.14±0.5°, 19.34±0.5°, 7.76±0.5°, and 28.73±0.5° in X-ray powder diffraction (XRD) analysis, or a dissolved solution thereof.

21. The saccharide syrup according to claim 20, wherein the saccharide syrup comprises the allulose dimer by 0.025 wt% or more, based on 100wt% of the solid content.

22. The saccharide syrup according to claim 20, wherein the content of the allulose dimer measured by gas chromatography (GC) analysis is 0.025 wt% or more.

23. The saccharide syrup according to claim 20, wherein the allulose dimer crystal has an X-ray powder diffraction pattern comprising peaks at positions of 2-theta diffraction angles of 15.51±0.5°, 17.66±0.5° and 18.14±0.5° in X-ray powder diffraction (XRD) analysis.

24. The saccharide syrup according to claim 20, wherein the allulose dimer crystal has an X-ray powder diffraction pattern comprising peaks at positions of 2-theta diffraction angles of 15.51±0.5°, 17.66±0.5°, 18.14±0.5°, and 19.34±0.5° in X-ray powder diffraction (XRD) analysis.

25. The saccharide syrup according to claim 20, wherein the allulose dimer crystal has an X-ray powder diffraction pattern comprising peaks at positions of 2-theta diffraction angles of 15.51±0.5°, 17.66±0.5°, 18.14±0.5°, 19.34±0.5° and 7.76±0.5° in X-ray powder diffraction (XRD) analysis.

26. The saccharide syrup according to claim 20, wherein the allulose dimer crystal has an X-ray powder diffraction pattern comprising peaks at positions of 2-theta diffraction angles of 15.51±0.5°, 17.66±0.5°, 18.14±0.5°, 19.34±0.5°, 7.76±0.5°, and 28.73±0.5° in X-ray powder diffraction (XRD) analysis.

27. The saccharide syrup according to claim 20, wherein the allulose dimer crystal has a melting point of 210±5°C.

28. The saccharide syrup according to claim 20, wherein the allulose dimer crystal has a solubility to water of 5 to 20 g/100g.

29. The saccharide syrup according to claim 20, wherein the allulose content is 20 to 99.975 wt%, based on 100 wt% of the solid content.

30. The saccharide syrup according to claim 20, wherein the solid content of the saccharide syrup is 20 to 90 wt%.

31. The saccharide syrup according to claim 20, wherein the allulose dimer is an agent of preventing or an agent of improving browning of allulose.

32. The saccharide syrup according to claim 20, wherein a yellowness change rate is -50% to 70%, when the syrup is stored under the condition of 60°C for 48 hours.

33. The saccharide syrup according to claim 20, wherein a moisture content after storing the syrup under the condition of 60°C for 24 hours is 57wt% or more based on 100wt% of the moisture content immediately after preparation.

34. The saccharide syrup according to claim 20, wherein the syrup further comprises at least one selected from the group consisting of monosaccharides other than allulose, disaccharides, sugar alcohols, oligosaccharides, high-intensity sweeteners, dietary fibers, proteins, vitamins, flavorings and colorants.

35. A food composition comprising the saccharide syrup according to any one of claims 20 to 34.

36. A method for preparation of a saccharide syrup comprising allulose, comprising a step of controlling a content of an allulose dimer crystal having an X-ray powder diffraction pattern comprising peaks at three or more positions selected from 2-theta diffraction angles of 15.51±0.5°, 17.66±0.5°, 18.14±0.5°, 19.34±0.5°, 7.76±0.5°, and 28.73±0.5° in X-ray powder diffraction (XRD) analysis or a dissolved solution thereof, in a saccharide syrup comprising allulose.

37. The method according to claim 36, wherein the step of controlling is controlling a content of the allulose dimer crystal or the dissolved solution thereof to 0.025wt% or more, based on 100wt% of the solid content of the saccharide syrup.

38. A method for preventing or improving browning of a saccharide syrup comprising allulose, comprising a step of controlling a content of an allulose dimer crystal having an X-ray powder diffraction pattern comprising peaks at three or more positions selected rom 2-theta diffraction angles of 15.51±0.5°, 17.66±0.5°, 18.14±0.5°, 19.34±0.5°, 7.76±0.5°, and 28.73±0.5° in X-ray powder diffraction (XRD) analysis or a dissolved solution thereof, in a saccharide syrup comprising allulose.

39. The method according to claim 38, wherein the step of controlling is controlling the content of the allulose dimer crystal or the dissolved solution thereof to 0.025wt% or more, based on 100wt% of the solid content of the saccharide syrup.

40. A method for preventing moisture loss of a saccharide syrup comprising allulose, comprising a step of controlling a content of an allulose dimer crystal having an X-ray powder diffraction pattern comprising peaks at three or more positions selected from 2-theta diffraction angles of 15.51±0.5°, 17.66±0.5°, 18.14±0.5°, 19.34±0.5°, 7.76±0.5°, and 28.73±0.5° in X-ray powder diffraction (XRD) analysis or a dissolved solution thereof, in a saccharide syrup comprising allulose.

41. The method according to claim 40, wherein the step of controlling is controlling the content of the allulose dimer crystal or the dissolved solution thereof to 1wt% or more, based on 100wt% of the solid content of the saccharide syrup.
